# EUROPÄISCHE PATENTSCHRIFT

(11) **EP 0 554 215 B1**
(45) Veröffentlichungstag und Bekanntmachung des Hinweises auf die Patenterteilung: **02.01.1997**
(21) Anmeldenummer: 93810029.4
(22) Anmeldetag: 19.01.1993
(51) Int. Cl.: C07C 69/54, G03F 7/027

(54) **Diacrylate und Dimethacrylate**
Diacrylates and dimethacrylates
Diacrylates et diméthacrylates

(30) Priorität: 27.01.1992 CH 216/92
(43) Veröffentlichungstag der Anmeldung: 04.08.1993
(73) Patentinhaber: CIBA-GEIGY AG, 4002 Basel (CH)
(72) Erfinder: Steinmann, Bettina, Dr., CH-1724 Praroman (CH); Schulthess, Adrian, Dr., CH-1734 Tentlingen (CH); Hunziker, Max, Dr., CH-3186 Düdingen (CH)

(56) Entgegenhaltungen:
- EP-A- 0 378 144
- EP-A- 0 425 441
- DE-A- 3 446 920
- CHEMICAL ABSTRACTS, vol. 116, 1992, Columbus, Ohio, US; abstract no. 49053x,

## Beschreibung

Die vorliegende Erfindung betrifft neue Acrylate und Methacrylate, photoempfindliche Zusammensetzungen enthaltend diese Verbindungen sowie ein Verfahren zur Herstellung von dreidimensionalen Gegenständen aus den photoempfindlichen Zusammensetzungen.

Strahlungsempfindliche flüssige Harze oder Harzsysteme können vielseitig eingesetzt werden, beispielsweise als Beschichtungsmittel, Klebstoffe oder Photoresists. Im Prinzip sollten flüssige Harze oder Harzsysteme auch für die Herstellung von dreidimensionalen Gegenständen nach dem im US-Patent 4,575,330 beschriebenen stereolithographischen Verfahren geeignet sein, doch erweisen sich manche Harze als zu viskos, andere wiederum sind zu wenig lichtempfindlich oder unterliegen beim Härten einem zu grossen Schrumpfungsprozess. Auch lassen die Festigkeitseigenschaften der Formkörper aus photogehärteten Harzen oft zu wünschen übrig.

In der EP-A 425441 werden flüssige Harzsysteme für die Stereolithographie beschrieben, enthaltend verschiedene Mono- und Diacrylate bzw. -methacrylate sowie ein Urethanarylat bzw. -methacrylat und ein monomeres oder oligomeres Diacrylat bzw. Methacrylat auf Basis von Bisphenol A oder Bisphenol F. Diese Gemische ergeben bei der Vorhärtung mittels Laserstrahlen Grünlinge mit hoher Grünfestigkeit und nach der Aushärtung hartelastische Gegenstände, deren Flexibilität jedoch für gewisse Anwendungen unzureichend ist.

Die EP-A 506616 offenbart flüssige Harzgemische aus mehreren Acrylaten bzw. Methacrylaten, die weitere hydroxygruppenhaltige aliphatische oder cycloaliphatische Acrylate bzw. Methacrylate enthalten. Die aus diesen Gemischen mittels Stereolithographie hergestellten ausgehärteten Formkörper zeichnen sich durch hohe Flexibilität und hohen Weiterreisswiderstand aus. Von Nachteil für die apparative Verarbeitung dieser Gemische ist jedoch die relativ hohe Viskosität.

Es wurden nun neue hydroxygruppenhaltige Acrylate und Methacrylate hergestellt, die in Kombination mit anderen Acrylaten oder Methacrylaten niedrigviskose photohärtbare Zusammensetzungen bilden und nach der vollständigen Aushärtung der Mischungen Formkörper mit ausgezeichneter Flexibilität liefern.

Gegenstand der vorliegenden Erfindung sind somit Verbindungen der Formeln (Ia) und (Ib) 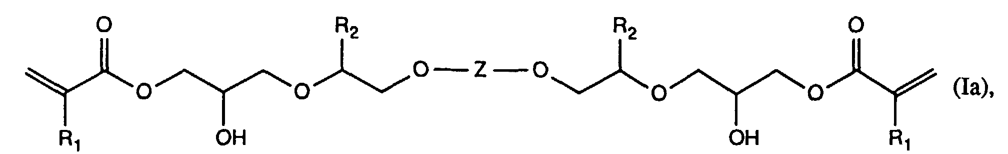 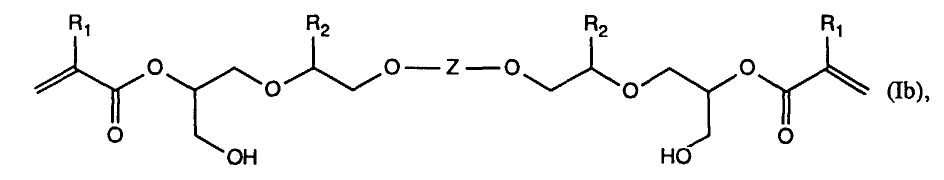 worin die Reste R₁ unabhängig voneinander Wasserstoff oder Methyl bedeuten,
R₂ für eine C₁-C₂₀-Alkylgruppe, die gegebenenfalls durch eine oder mehrere Hydroxygruppen, C₆-C₁₄-Arylgruppen oder Halogenatome substituiert ist, eine unsubstituierte oder durch eine oder mehrere C₁-C₆-Alkylgruppen, Hydroxygruppen oder Halogenatome substituierte Phenylgruppe oder für einen Rest der Formel -CH₂-OR₃ steht, worin R₃ eine C₁-C₂₀-Alkylgruppe, die gegebenenfalls durch eine oder mehrere Hydroxygruppen, C₆-C₁₄-Arylgruppen oder Halogenatome substituiert ist, eine unsubstituierte oder durch eine oder mehrere C₁-C₆-Alkylgruppen, Hydroxygruppen oder Halogenatome substituierte Phenylgruppe, eine C₂-C₆-Alkenylgruppe, eine C₂-C₂₀-Acylgruppe oder eine unsubstituierte oder durch eine oder mehrere C₁-C₆-Alkylgruppen, Hydroxygruppen oder Halogenatome substituierte Cyclohexylcarbonylgruppe bedeutet,
Z eine Gruppe der Formeln (IIa)-(IIe) ist, 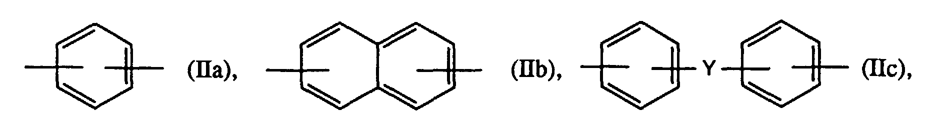 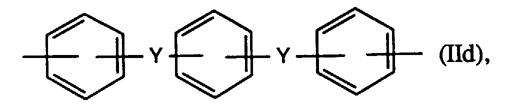 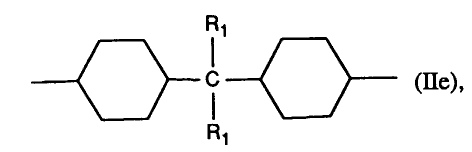 worin Y für eine direkte Bindung, C₁-C₆-Alkylen, -S-, -O-, -SO-, -SO₂- oder -CO- steht und R₁ Wasserstoff oder Methyl bedeutet und worin die aromatischen bzw. cycloaliphatischen Ringe der Formeln (IIa)-(IIe) unsubstituiert oder durch eine oder mehrere C₁-C₆-Alkylgruppen, Chlor- oder Bromatome substituiert sind.

Steht R₂ oder R₃ für eine C₁-C₂₀-Alkylgruppe, so kann es sich dabei um verzweigte oder insbesondere um geradkettige Alkylgruppen handeln.
Beispiele für solche Alkylgruppen sind Methyl, Ethyl, n-Propyl, i-Propyl, n-Butyl, sek.-Butyl, tert.-Butyl, n-Pentyl, neo-Pentyl, n-Hexyl, Octyl, Decyl, Dodecyl und Eicosyl.

Die Alkylgruppen können auch durch eine oder mehrere Hydroxygruppen, C₆-C₁₄-Arylgruppen oder Halogenatome substituiert sein. Beispiele für substituierte Alkylgruppen sind Hydroxymethyl, 2-Hydroxyethyl, 2-Hydroxypropyl, 2-Chlorpropyl, 2,3-Dichlorbutyl, 2-Phenylethyl und 2,3-Diphenylbutyl.

Bedeutet R₃ C₂-C₆-Alkenyl, so handelt es sich dabei um verzweigte oder insbesondere um geradkettige Reste. Beispiele für Alkenylreste sind Vinyl, Prop-1-enyl, Prop-2-enyl, 2-Methylprop-2-enyl, n-But-3-enyl, n-Pent-4-enyl und n-Hex-5-enyl. Bevorzugt werden geradkettige Alkenylreste mit zwei oder drei Kohlenstoffatomen, insbesondere Vinyl, Prop-1-enyl und Prop-2-enyl.

Beispiele für C₂-C₂₀-Acylgruppen sind Acetyl, Propionyl, n-Butyryl, i-Butyryl, Pivaloyl, Hexyloyl, Octyloyl, Tetradecyloyl, Hexadecyloyl und Octadecyloyl.

R₃ als Phenyl oder Cyclohexylcarbonyl kann unsubstituiert oder durch eine oder mehrere C₁-C₆-Alkylgruppen, Hydroxygruppen oder Halogenatome substitiert sein.
Beispiele dafür sind Tolyl, Xylyl, Mesityl, 2-Hydroxyphenyl, 4-Hydroxyphenyl, 2-Chlorphenyl, 4-Chlorphenyl, 3,5-Dichlorphenyl, 2,4-Dichlorphenyl, 2,6-Dimethylcyclohexylcarbonyl, 4-Hydroxycyclohexylcarbonyl, p-Hydroxybenzyl, p-Chlorbenzyl und o-Ethylbenzyl.

Die aromatischen bzw. cycloaliphatischen Ringe in den Formeln (IIa)-(IIe) sind vorzugsweise unsubstituiert.

In den Verbindungen der Formeln (Ia) und (Ib) steht R₂ bevorzugt für C₁-C₂₀-Alkyl, Phenyl, C₁-C₂₀-Alkoxymethyl, Phenoxymethyl oder Cyclohexylcarbonyloxymethyl.

Besonders bevorzugt sind Verbindungen der Formel (Ia) und (Ib), worin R₂ n-Butyl, Phenyl, n-Butoxymethyl, Phenoxymethyl oder Cyclohexylcarbonyloxymethyl bedeutet.

Ganz besonders bevorzugt steht R₂ für n-Butoxymethyl.

Z steht in den Formeln (Ia) und (Ib) vorzugsweise für eine Gruppe der Formeln (IIc) oder (IIe).

Besonders bevorzugt sind Verbindungen der Formeln (Ia) und (Ib), worin Z 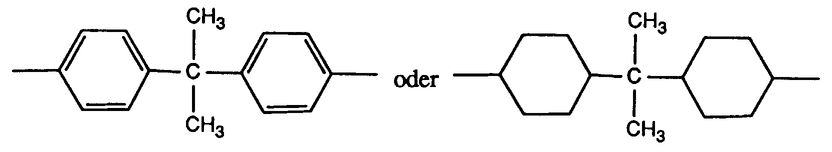 bedeutet.

Die Verbindungen der Formeln (Ia) und (Ib) können nach an sich bekannten Verfahren hergestellt werden. Ein weiterer Erfindungsgegenstand ist ein Verfahren zur Herstellung von Verbindungen der Formeln (Ia) und (Ib), dadurch gekennzeichnet, dass man einen Diglycidylether der Formel (III) 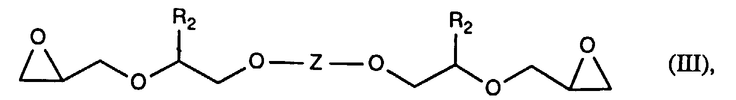 worin R₂ und Z die oben angegebene Bedeutung haben, in an sich bekannter Weise mit Acrylsäure oder Methacrylsäure umsetzt.

Die Diglycidylverbindungen der Formel (III) sind bekannt und beispielsweise in der EP-A 22073 beschrieben.
Bei der Umsetzung der Diglycidylverbindungen Der Formel (III) mit Acryl- oder Methacrylsäure entsteht in der Regel ein Gemisch der Verbindungen (Ia) und (Ib), wobei die Verbindung (Ia) das Hauptprodukt darstellt und die Verbindung (Ib) in vergleichsweise geringen Mengen (ca. 10-20 %) anfällt. Für die Verwendung der erfindungsgemässen Verbindungen in photoempfindlichen Zusammensetzungen ist eine Trennung der beiden strukturisomeren Verbindungen nicht erforderlich.

Als spezifische Beispiele für die Diglycidylverbindungen der Formel (Ill) seien genannt:
2,2-Bis-[p-(3-butoxy-2-glycidyloxypropyloxy)phenyl]-propan,
2,2-Bis-[p-(3-methoxy-2-glycidyloxypropyloxy)phenyl]-propan,
2,2-Bis-[p-(3-ethoxy-2-glycidyloxypropyloxy)phenyl]-propan,
2,2-Bis-[p-(3-dodecyloxy-2-glycidyloxypropyloxy)phenyl]-propan,
2,2-Bis-[p-(3-tetradecyloxy-2-glycidyloxypropyloxy)phenyl]-propan,
2,2-Bis-[p-(3-benzyloxy-2-glycidyloxypropyloxy)phenyl]-propan,
Bis-[p-(3-butoxy-2-glycidyloxypropyloxy)phenyl]-methan,
1,3-Bis-[p-(3-phenoxy-2-glycidyloxypropyloxy]-benzol
Bis-[p-(3-butoxy-2-glycidyloxypropyloxy)phenyl]-sulfon,
2,2-Bis-[p-(3-cyclohexyloxy-2-glycidyloxypropyloxy)phenyl]-propan,
2,2-Bis-[4(3-butoxy-2-glycidyloxypropyloxy)-3,5-dibromphenyl]-propan,
2,2-Bis-[p-(3-allyloxy-2-glycidyloxypropyloxy)phenyl]-propan,
2,2-Bis-[p-(3-phenoxy-2-glycidyloxypropyloxy)phenyl]-propan,
2,2-Bis-[4-(3-butoxy-2-glycidyloxypropyloxy)cyclohexyl]-propan,
2,2-Bis- [p-(3-cyclohexylcarbonyloxy-2-glycidyloxypropyloxy)phenyl]-propan,
2,2-Bis-[p-(2-glycidyloxyhexyloxy)phenyl]-propan und
2,2-Bis-[p-(2-phenyl-2-glycidyloxyethoxy)phenyl]-propan.

Einen weiteren Erfindungsgegenstand bildet eine photoempfindliche Zusammensetzung enthaltend
(a) 5-65 Gew.-% einer Verbindung der Formel (Ia) oder (Ib) gemäss Anspruch 1,
(b) 15-70 Gew.-% eines oder mehrerer von der Verbindung der Formel (Ia) oder (Ib) verschiedenen bifunktionellen Acrylate oder Methacrylate mit einem Molekulargewicht zwischen 150 und 450,
(c) 0-40 Gew.-% eines oder mehrerer monomerer polyfunktioneller Acrylate oder Methacrylate mit einer Funktionalität von mindestens 3 und einem Molekulargewicht von höchstens 600,
(d) 0-10 Gew.-% mindestens eines monofunktionellen Acrylats oder Methacrylats,
(e) 0-10 Gew.-% N-Vinylpyrrolidon oder N-Vinylcaprolactam,
(f) 2-10 Gew.-% mindestens eines Photoinitiators und
(g) 0-60 Gew.-% mindestens eines Urethanacrylats oder -methacrylats mit einer Funktionalität von 2-4 und einem Molekulargewicht von 500-10000,
wobei die Summe der Anteile der Komponenten (a) bis (g) zusammen 100 Gew.-% beträgt.

Als Verbindungen der Komponente (b) eignen sich beispielsweise die Diacrylat- und Dimethacrylatester von aliphatischen, cycloaliphatischen oder aromatischen Diolen, wie 1,3- oderl,4-Butandiol, Neopentylglykol, 1,6-Hexandiol, Diethylenglykol, Triethylenglykol, Tetraethylenglykol, Polyethylenglykol, Tripropylenglykol, ethoxyliertes oder propoxyliertes Neopentylglykol, 1,4-Dihydroxymethylcyclohexan, 2,2-Bis-(4-hydroxycyclohexyl)-propan, Bis-(4-hydroxycyclohexyl)-methan, Hydrochinon, 4,4'-Dihydroxybiphenyl, Bisphenol A, Bisphenol F, Bisphenol S, ethoxyliertes oder propoxyliertes Bisphenol A, ethoxyliertes oder propoxyliertes Bisphenol F oder ethoxyliertes oder propoxyliertes Bisphenol S.

Solche Diacrylate und Dimethacrylate sind bekannt und zum Teil im Handel erhältlich, beispielsweise unter den Produktbezeichnungen SR-348 für ethoxyliertes Bisphenol A-dimethacrylat, SR-349 für ethoxyliertes Bisphenol A-diacrylat, SR 247 für Neopentylglykoldiacrylat und SR 344 für Polyethylenglykol-400-diacrylat von der Firma SARTOMER Company.

Vorzugsweise wird als Komponente (b) ein Diacrylat oder Dimethacrylat von ethoxyliertem Bisphenol A verwendet.

Als Verbindungen der Komponente (c) eignen sich beispielsweise Triacrylate oder -methacrylate der Formeln (IV) oder (V)

R₄-CH₂-C(̵CH₂ -R₅)₃ (IV),

R₅-CH(̵CH₂-R₅)₂ (V),

worin R₄ ein Wasserstoffatom, Methyl oder Hydroxyl bedeutet und R₅ einen Rest der Formel (VI) darstellt 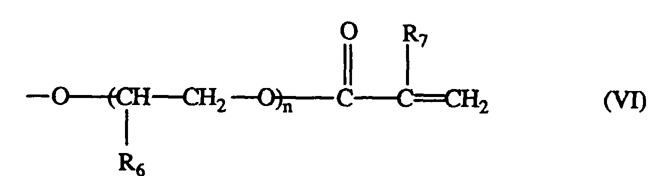 worin n für Null oder eine Zahl von 1-3 steht und R₆ und R₇ unabhängig voneinander je ein Wasserstoffatom oder Methyl bedeuten.

Von den Verbindungen der Formeln (IV) und (V) sind solche der Formel (IV), worin R₄ eine Methylgruppe und R₅ einen Rest der Formel (VI) bedeuten, worin n Null ist, besonders bevorzugt.

Als Komponente (c) können beispielsweise eingesetzt werden:
1,1,1-Trimethylolpropantriacrylat oder -methacrylat, ethoxyliertes oder propoxyliertes 1,1,1-Trimethylolpropantriacrylat oder -methacrylat, ethoxyliertes oder propoxyliertes Glycerintriacrylat, Pentaerythritolmonohydroxytriacrylat oder -methacrylat; auch höherfunktionelle Acrylate oder Methacrylate wie z.B. Dipentaerythritolmonohydroxypentaacrylat oder Bistrimethylolpropantetraacrylat. Solche Verbindungen sind dem Fachmann bekannt und zum Teil im Handel erhältlich.

Insbesondere weisen die Verbindungen der Komponente (c) ein Molekulargewicht von 250 bis 700 auf.

Besonders bevorzugt als Komponente (c) sind Trimethylolpropantriacrylat und Trimethylolpropantrimethacrylat.

In den erfindungsgemässen Gemischen können gegebenenfalls als Komponente (d) beispielsweise folgende Verbindungen enthalten sein: Allylacrylat, Allylmethacrylat, Methyl-, Ethyl-, n-Propyl-, n-Butyl-, Isobutyl-, n-Hexyl-, 2-Ethylhexyl-, n-Octyl-, n-Decyl- und n-Dodecylacrylat und -methacrylat, 2-Hydroxyethyl-, 2- und 3-Hydroxypropylacrylat und -methacrylat, 2-Methoxyethyl-, 2-Ethoxyethyl- und 2- oder 3-Ethoxypropylacrylat, Tetrahydrofurfurylmethacrylat, 2-(2-Ethoxyethoxy)ethylacrylat, Cyclohexylmethacrylat, 2-Phenoxyethylacrylat, 2-Phenoxyethylmethacrylat, Glycidylacrylat und Isodecylacrylat. Solche Produkte sind ebenfalls bekannt und zum Teil im Handel erhältlich, zum Beispiel von der Firma SARTOMER Company.

Besonders bevorzugt ist 2-Phenoxyethylacrylat.

Die erfindungsgemässen Zusammensetzungen können gegebenenfalls als Komponente (e) bis zu 10 Gew.-% N-Vinylpyrrolidon oder N-Vinylcaprolactam oder Gemische davon enthalten. Vorzugsweise wird N-Vinylpyrrolidon eingesetzt.

In den erfindungsgemässen Zusammensetzungen können alle Typen von Photoinitiatoren als Komponente (f) eingesetzt werden, welche bei der entsprechenden Bestrahlung freie Radikale bilden. Typische bekannte Photoinitiatoren sind Benzoine und Benzoinether, wie Benzoin, Benzoinmethylether, Benzoinethylether und Benzoinisopropylether, Benzoinphenylether und Benzoinacetat; Acetophenone, wie Acetophenon, 2,2-Dimethoxyacetophenon, und 1,1-Dichloracetophenon; Benzil, Benzilketale, wie Benzildimethylketal und Benzildiethylketal; Anthrachinone, wie 2-Methylanthrachinon, 2-Ethylanthrachinon, 2-tert.-Butylanthrachinon, 1-Chloranthrachinon und 2-Amylanthrachinon; Triphenylphosphin; Benzoylphosphinoxide, wie beispielsweise 2,4,6-Trimethylbenzoyldiphenylphosphinoxid (Luzirin TPO); Benzophenone, wie Benzophenon und 4,4'-Bis-(N,N'-dimethylamino)-benzophenon; Thioxanthone und Xanthone; Acridinderivate; Phenazinderivate; Quinoxalinderivate oder 1-Phenyl-1,2-propandion-2-O-benzoyloxim; 1-Aminophenylketone oder 1-Hydroxyphenylketone, wie 1-Hydroxycyclohexylphenylketon, Phenyl-(1-hydroxyisopropyl)-keton und 4-Isopropylphenyl(1-hydroxyisopropyl)-keton.

Geeignet sind auch Elektronentransfer-Initiatoren vom Typ der Xanthone, wie beispielsweise 2,4,5,7-Tetraiodo-6-hydroxy-9-cyano-3H-xanthen-3-on, die zusammen mit geeigneten Elektronendonatoren im sichtbaren Bereich des Spektrums eine hohe Reaktivität aufweisen.

Eine weitere Klasse von geeigneten Photoinitiatoren (f) stellen die ionischen Farbstoff-Gegenionverbindungen (ionic dye-counter ion compound) dar, welche in der Lage sind, aktinische Strahlen zu absorbieren und freie Radikale zu erzeugen, die die Polymerisation der Acrylate (a) bis (d) und gegebenenfalls (g) initiieren. Die erfindungsgemässen Gemische, welche ionische Farbstoff-Gegenionverbindungen enthalten, können auf diese Weise mit sichtbarem Licht im einstellbaren Wellenlängenbereich von 400-700 nm variabler gehärtet werden. Ionische Farbstoff-Gegenionverbindungen und deren Wirkungsweise sind bekannt, beispielsweise aus der EP-A-O 223 587 und den US-Patenten 4,751,102; 4,772,530 und 4,772,541. Als Beispiele für geeignete ionische Farbstoff-Gegenionverbindungen seien genannt die anionischen Farbstoff-Iodoniumionkomplexe, die anionischen Farbstoff-Pyrylliumionkomplexe und insbesondere die kationischen Farbstoff-Boratanionverbindungen der folgenden Formel 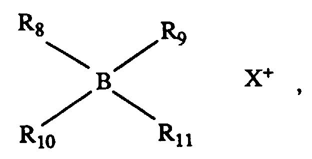 worin X⁺ für einen kationischen Farbstoff steht und R₈, R₉, R₁₀ und R₁₁ unabhängig voneinander je ein Alkyl, Aryl, Alkaryl, Allyl, Aralkyl, Alkenyl, Alkinyl, eine alicyclische oder gesättigte oder ungesättigte heterocyclische Gruppe bedeuten.

Besonders geeignete Photoinitiatoren, welche gewöhnlich in Kombination mit einem HeCd-Laser als Strahlenquelle verwendet werden, sind Acetophenone, wie 2,2-Dialkoxybenzophenone, und α-Hydroxyphenylketone, beispielsweise 1-Hydroxycyclohexylphenylketon oder (2-Hydroxyisopropyl)-phenylketon (= 2-Hydroxy-2,2-dimethylacetophenon).

Ein besonders bevorzugter Photoinitiator ist 1-Hydroxycyclohexylphenylketon.

Die erfindungsgemässen Gemische können auch verschiedene Photoinitiatoren enthalten, welche gegenüber Strahlen von Emissionslinien unterschiedlicher Wellenlängen verschieden strahlungsempfindlich sind. Man erreicht dadurch beispielsweise eine bessere Ausnützung einer UV/VIS-Lichtquelle, welche Emissionslinien unterschiedlicher Wellenlänge ausstrahlt. Vorteilhaft ist es dabei, wenn die verschiedenen Photoinitiatoren so ausgewählt und in einer solchen Konzentration eingesetzt werden, dass bei den verwendeten Emissionslinien eine gleiche optische Absorption erzeugt wird.

Die in den erfindungsgemässen Gemischen als Komponente (g) verwendeten Urethanacrylate sind dem Fachmann bekannt und können in bekannter Weise hergestellt werden, indem man beispielsweise ein hydroxylterminiertes Polyurethan mit Acrylsäure oder Methacrylsäure zu dem entsprechenden Urethanacrylat umsetzt, oder indem man ein isocyanatterminiertes Präpolymer mit Hydroxyalkylacrylaten oder -methacrylaten zum Urethanacrylat umsetzt. Entsprechende Verfahren werden beispielsweise in den EP-A 114982 und EP-A 133908 offenbart. Das Molekulargewicht solcher Acrylate liegt im allgemeinen im Bereich von 400 bis 10000, vorzugsweise zwischen 500 bis 7000. Urethanacrylate sind auch im Handel erhältlich und werden beispielsweise unter der Bezeichnung EBECRYL® von der Firma UCB, unter der Bezeichnung Uvithane® von der Firma Morton Thiokol oder unter den Produktebezeichnungen SR 9504, SR 9600, SR 9610, SR 9620, SR 9630, SR 9640 und SR 9650 von der Firma SARTOMER Company angeboten.

Bevorzugt werden als Urethanacrylate solche eingesetzt, die ein MG von 500-7000 aufweisen und die insbesondere aus aliphatischen Edukten hergestellt worden sind.

Die erfindungsgemässen, photoempfindlichen Gemische können durch Bestrahlen mit aktinischem Licht, beispielsweise mittels Elektronen-, Röntgenstrahlen, UV- oder VIS-Licht, das heisst, mit Strahlen im Wellenlängenbereich von 280-650 nm, polymerisiert werden. Besonders geeignet sind Laserstrahlen von HeCd, Argon oder Stickstoff sowie Metalldampf und NdYAG-Laser mit vervielfachter Frequenz. Es ist dem Fachmann bekannt, dass für jede gewählte Lichtquelle der geeignete Photoinitiator ausgewählt und gegebenenfalls sensibilisiert werden muss. Man hat erkannt, dass die Eindringtiefe der Strahlen in die zu polymerisierende Zusammensetzung und die Arbeitsgeschwindigkeit in direktem Zusammenhang mit dem Absorptionskoeffizienten und der Konzentration des Photoinitiators stehen. In der Stereolithographie werden vorzugsweise solche Photoinitiatoren eingesetzt, welche die höchste Anzahl von entstehenden freien Radikalen bewirken und die grösste Strahlungseindringtiefe in die zu polymerisierenden Zusammensetzungen ermöglichen.

Ein weiterer Erfindungsgegenstand ist ein Verfahren zur Herstellung von dreidimensionalen Gegenständen aus den flüssigen, erfindungsgemässen Zusammensetzungen mittels Stereolithographie, wobei die Oberfläche einer Schicht aus dem erfindungsgemässen, flüssigen Gemisch ganzflächig oder in einem vorbestimmten Muster mit UV- oder VIS-Licht bestrahlt wird, so dass sich in den bestrahlten Bereichen eine Schicht in einer gewünschten Schichtdicke verfestigt, dann eine neue Schicht aus den erfindungsgemässen Gemischen auf der verfestigten Schicht gebildet wird, die ebenfalls ganzflächig oder in einem vorbestimmten Muster bestrahlt wird, und wobei durch wiederholtes Beschichten und Bestrahlen dreidimensionale Gegenstände aus mehreren aneinander haftenden, verfestigten Schichten erhalten werden.

Bei diesen Verfahren wird als Strahlungsquelle bevorzugt ein Laserstrahl verwendet, der vorzugsweise computergesteuert ist.

Die erfindungsgemässen Zusammensetzungen zeichnen sich durch niedrige Viskosität und damit gute Verarbeitbarkeit aus. Die durch Vorhärtung mittels Laserstrahlen erhaltenen Grünlinge und die vollständig ausgehärteten Gegenstände besitzen gute mechanische Eigenschaften, insbesondere eine hohe Flexibifität.

Die erfindungsgemässen Zusammensetzungen können beispielsweise als Klebstoffe, Beschichtungsmittel oder als Formulierungen für die Stereolithographie oder andere Verfahren für den Modellbau mit Photopolymeren verwendet werden.

Werden die erfindungsgemässen Gemische als Beschichtungsmittel eingesetzt, so erhält man klare und harte Beschichtungen auf Holz, Papier, Metall, Keramik oder anderen Oberflächen. Die Beschichtungsdicke kann dabei sehr variieren und etwa von 1 µm bis etwa 1 mm betragen. Aus den erfindungsgemässen Gemischen können Reliefbilder für gedruckte Schaltungen oder Druckplatten direkt durch Bestrahlen der Gemische hergestellt werden, beispielsweise mittels eines computergesteuerten Laserstrahls geeigneter Wellenlänge oder unter Anwendung einer Photomaske und einer entsprechenden Lichtquelle.

Vorzugsweise verwendet man die erfindungsgemässen Zusammensetzungen zur Herstellung von photopolymerisierten Schichten, insbesondere in Form von dreidimensionalen Gegenständen, die aus mehreren aneinander haftenden, verfestigten Schichten aufgebaut sind.

### Beispiele:

### I. Herstellung der neuen Acrylate und Methacrylate

### I.1. Acrylat A: Diacrylat des 2,2-Bis-[p-(3-butoxy-2-glycidyloxypropyloxy)phenyl]-propans

Methode I: 100 g 2,2-Bis-[p-(3-butoxy-2-glycidyloxypropyloxy)phenyl]-propan (hergestellt gemäss EP-A 22073) mit einem Epoxidgehalt von 2,9 val/kg werden in 250 ml Toluol gelöst. Nach Zugabe von 1 g Tetraethylammoniumbromid und 0,2 g Hydrochinonmonomethylether wird die Mischung auf 80 °C erhitzt. Anschliessend wird ein Gemisch aus 22,98 g (0,32 mol) Acrylsäure und 0,17 g Hydrochinonmonomethylether langsam zugetropft. Das Reaktionsgemisch wird so lange auf 80 °C gehalten, bis der Epoxidgehalt weniger als 0,1 val/kg beträgt (ca. 14 h). Dann wird auf Raumtemperatur abgekühlt und mit einer 5%igen wässrigen NaHCO₃-Lösung und anschliessend mit Wasser ausgeschüttelt. Die organische Phase wird getrocknet und zunächst im Rotationsverdampfer und anschliessend im Hochvakuum eingeengt.
Ausbeute: 106,94 g (88,5 %)

Methode II: 343,9 g 2,2-Bis-[p-(3-butoxy-2-glycidyloxypropyloxy)phenyl]-propan (hergestellt gemäss EP-A 22073) mit einem Epoxidgehalt von 2,9 val/kg werden mit 0,2 g Di-tert.-butyl-p-kresol versetzt und auf 110 °C erhitzt. Unter Rühren wird ein Gemisch aus 72,06 g (1 mol) Acrylsäure, 0,56 g Nuosyn Chromium® 5% (Fettsäure-Chromsalz in Kohlenwasserstoffen der Fa. Durham Chemicals, GB) und 0,42 g Di-tert.-butyl-p-kresol zugetropft. Das Gemisch wird bei 110 °C gehalten, bis der Epoxidgehalt weniger als 0,1 val/g beträgt (ca. 4 h). Es wird ein bräunliches viskoses Harz mit einem Doppelbindungsgehalt von 2,38 val/kg (88,5 % der Theorie) erhalten.

### I.2. Methacrylat B: Dimethacrylat des 2,2-Bis-[p-(3-butoxy-2-glycidyloxypropyloxy)phenyl]-propans

343,9 g 2,2-Bis-[p-(3-butoxy-2-glycidyloxypropyloxy)phenyl]-propan (hergestellt gemäss EP-A 22073) mit einem Epoxidgehalt von 2,9 val/kg werden mit 86,09 g (1 mol) Methacrylsäure nach der oben beschriebenen Methode II umgesetzt. Die Mischung wird bei ca. 110 °C gerührt, bis der Epoxidgehalt weniger als 0,1 val/kg beträgt (ca. 4 h). Es wird ein bräunliches viskoses Harz mit einem Doppelbindungsgehalt von 2,26 val/kg (87,2 % der Theorie) erhalten.

### I.3. Acrylat C: Diacrylat des 2,2-Bis-[4-(3-butoxy-2-glycidyloxypropyloxy)cyclohexyl]-propans

100 g 2,2-Bis-[4-(3-butoxy-2-glycidyloxypropyloxy)cyclohexyl]-propan (hergestellt gemäss EP-A 22073) mit einem Epoxidgehalt von 2,48 val/kg werden mit 19,67 g (0,273 mol) Acrylsäure nach der oben beschriebenen Methode I umgesetzt. Die Lösung wird 4 h bei 80 °C gerührt. Der Epoxidgehalt beträgt dann noch 0,12 val/kg. Nach Ausschütteln mit 5%iger wässriger NaHCO₃-Lösung und anschliessend mit Wasser wird die organische Phase im Hochvakuum eingeengt.
- Ausbeute:: 87,8 g (74,5 %)
- Doppelbindungsgehalt:: 1,91 val/kg (71,8 % der Theorie)

### I.4. Methacrylat D: Dimethacrylat des 2,2-Bis-[4-(3-butoxy-2-glycidyloxypropyloxy)cyclohexyl]-propans

82 g 2,2-Bis-[4-(3-butoxy-2-glycidyloxypropyloxy)cyclohexyl]-propan (hergestellt gemäss EP-A 22073) mit einem Epoxidgehalt von 2,48 val/kg werden mit 19,2 g (0,223 mol) Methacrylsäure nach der oben beschriebenen Methode I umgesetzt. Die Lösung wird ca. 32 h bei 80 °C gerührt. Der Epoxidgehalt beträgt dann noch 0, 17 val/kg. Nach Ausschütteln mit 5%iger wässriger NaHCO₃-Lösung und anschliessend mit Wasser wird die organische Phase im Hochvakuum eingeengt.
- Ausbeute:: 84,33 g (84,7 %)
- Doppelbindungsgehalt:: 1,90 val/kg (74,5 % der Theorie)

### I.5. Acrylat E: Diacrylat des 2,2-Bis-[p-(3-phenyloxy-2-glycidyloxypropyloxy)phenyl]-propans

50 g 2,2-Bis-[p-(3-phenyloxy-2-glycidyloxypropyloxy)phenyl]-propan (hergestellt gemäss EP-A 22073) mit einem Epoxidgehalt von 2,7 val/kg werden mit 9,76 g (0,135 mol) Acrylsäure nach der oben beschriebenen Methode II umgesetzt. Man erhält ein viskoses Harz mit einem Doppelbindungsgehalt von 2,28 val/kg (85,8 % der Theorie).

### I.6. Acrylat F: Diacrylat des 2,2-Bis-[p-(3-cyclohexylcarbonyloxy-2-glycidyloxypropyloxy)phenyl]-propans

a) Herstellung von 2,2-Bis-[p-(3-cyclohexylcarbonyloxy-2-glycidyloxypropyloxy)phenyl]-propan (nach EP-A 22073):
   50 g (0,27 mol) Cyclohexansäureglycidylester werden mit 30 g (0,135 mol) Bisphenol A und 0,8 g Benzyltrimethylammoniumbromid unter Rühren auf 110 °C erhitzt. Nach Abklingen der exothermen Reaktion (Temperaturanstieg auf 140 °C) wird das Reaktionsgemisch weiter bei 110 °C gerührt, bis der Epoxidgehalt weniger als 0,1 val/kg beträgt (2 h).
   35 g (0,059 mol) des so erhaltenen Reaktionsprodukts werden mit 87 g (0,94 mol) Epichlorhydrin und 0,77 g Tetramethylammoniumbromid nach der in der EP-A 22073 beschriebenen Methode umgesetzt. Nach Zugabe von 9,6 g (0,12 mol) 50%iger Natronlauge und Entfernen des Wassers im Vakuum wird das Produkt isoliert und getrocknet.
   - Ausbeute:: 15,5 g (37 %)
   - Epoxidgehalt:: 1,90 val/kg (74,5 % der Theorie)
b) Herstellung des Diacrylats:
   12,97 g (0,27 mol) des nach a) hergestellten 2,2-Bis-[p-(3-cyclohexylcarbonyloxy-2-glycidyloxypropyloxy)phenyl]-propans werden mit 1,8 g (0,025 mol) Acrylsäure nach der oben beschriebenen Methode II umgesetzt. Es wird ein viskoses Harz mit einem Doppelbindungsgehalt von 1,84 val/kg (78,3 % der Theorie) erhalten.

### I.7. Acrylat G: Diacrylat des 2,2-Bis-[p-(2-glycidyloxyhexyloxy)phenyl]-propans

a) Herstellung von 2,2-Bis-[p-(2-glycidyloxyhexyloxy)phenyl]-propan (nach EP-A 22073):
   100,2 g (1 mol) Butyloxiran werden mit 114 g (0,5 mol) Bisphenol A und 2,14 g Benzyltrimethylammoniumbromid unter Rühren auf 110 °C erhitzt. Nach Abklingen der exothermen Reaktion (Temperaturanstieg auf 115 °C) wird das Reaktionsgemisch weiter bei 110 °C gerührt, bis der Epoxidgehalt weniger als 0,1 val/kg beträgt (16 h). 85,52 g (0,2 mol) des so erhaltenen Reaktionsprodukts werden mit 296 g (3,2 mol) Epichlorhydrin und 1,32 g Tetramethylammoniumbromid nach der in der EP-A 22073 beschriebenen Methode umgesetzt. Nach Zugabe von 33,6 g (0,42 mol) 50%iger Natronlauge und Entfernen des Wassers im Vakuum wird das Produkt isoliert und getrocknet.
   - Ausbeute:: 87,2 g (80,6 %)
   - Epoxidgehalt:: 2,51 val/kg (67,9% der Theorie)
b) Herstellung des Diacrylats:
   100 g (0,11 mol) des nach a) hergestellten 2,2-Bis-[p-(2-glycidyloxyhexyloxy)phenyl]-propans werden mit 15,85 g (0,22 mol) Acrylsäure nach der oben beschriebenen Methode II umgesetzt. Es wird ein viskoses Harz mit einem Doppelbindungsgehalt von 1,87 val/kg (64 % der Theorie) erhalten.

### I.8. Acrylat H: Diacrylat des 2,2-Bis-[p-(2-phenyl-2-glycidyloxyethoxy)phenyl]-propans

a) Herstellung von 2,2-Bis-[p-(2-phenyl-2-glycidyloxyethoxy)phenyl]-propan (nach EP-A 22073):
   100 g (0,83 mol) Phenylethylenoxid werden mit 94,7 g (0,415 mol) Bisphenol A und
   1,95 g Benzyltrimethylammoniumbromid unter Rühren auf 110 °C erhitzt. Nach Abklingen der exothermen Reaktion (Temperaturanstieg auf 115 °C) wird das Reaktionsgemisch weiter bei 110 °C gerührt, bis der Epoxidgehalt weniger als 0,1 val/kg beträgt (5 h).
   80 g (0,17 mol) des so erhaltenen Reaktionsprodukts werden mit 251,6 g (2,72 mol)
   Epichlorhydrin und 0,9 g Tetramethylammoniumchlorid nach der in der EP-A 22073 beschriebenen Methode umgesetzt. Nach Zugabe von 28,8 g (0,36 mol) 50%iger Natronlauge und Entfernen des Wassers im Vakuum wird das Produkt isoliert und getrocknet.
   - Ausbeute:: 60,5 g (58,5 %)
   - Epoxidgehalt:: 2,56 val/kg (78 % der Theorie)
b) Herstellung des Diacrylats:
   25 g (0,032 mol) des nach a) hergestellten 2,2-Bis-[p-(2-phenyl-2-glycidyloxyethoxy)phenyl]-propans werden mit 4,6 g (0,064 mol) Acrylsäure nach der oben beschriebenen Methode II umgesetzt. Es wird ein viskoses Harz mit einem Doppelbindungsgehalt von 2, 12 val/kg (76,8 % der Theorie) erhalten.

### II. Anwendungsbeispiele Verwendung der neuen Diacrylate und Dimethacrylate in Mischungen für die Stereolithographie

### Beispiel 1:

49,85 g Acrylat A, 26 g Ethoxyliertes Bisphenol-A-dimethacrylat (SR 348 der Fa. Sartomer), 14 g Trimethylolpropantrimethacrylat (SR 350, Sartomer) und 6 g Phenoxyethylacrylat (SR 339, Sartomer) werden bei ca. 60 °C mit 0, 15 g Hydrochinonmonomethylether und 4 g 1-Hydroxycyclohexylphenylketon vermischt. Die resultierende homogene flüssige Mischung weist eine Viskosität von 631 mPa·s bei 30 °C auf. Ein aus diesem Gemisch mit Hilfe eines He/Cd-Lasers (40 mJ/cm²) gehärteter Formkörper (Grünling) weist einen Elastizitätsmodul (DIN 53371; green strength) von 16,2 N/mm², eine Zugfestigkeit σₘₐₓ (DIN 53455) von 1,31 N/mm² und eine Bruchdehnung ε (DIN 53455) von 10,2 % auf.
Zur vollständigen Aushärtung wird der Grünling 30 min mit UV/VIS-Licht bestrahlt. Der Formkörper weist anschliessend folgende Eigenschaften auf:

| | |
|---|---|
| Elastizitätsmodul: | 1610 N/mm² |
| Zugfestigkeit σₘₐₓ: | 32,8 N/mm² |
| Bruchdehnung ε: | 7,2 % |

### Beispiele 2-10:

Es werden wie in Beispiel 1 Formulierungen aus den in den Tabellen 1 und 2 angegebenen Komponenten hergestellt und zu dreidimensionalen Formkörpern verarbeitet. Die Eigenschaften der flüssigen Mischungen, der Grünlinge und der ausgehärteten Formkörper sind ebenfalls in den Tabellen 1 und 2 angegeben.

**Tabelle 1:**

| Beispiel | 1 | 2 | 3 | 4 |
|---|---|---|---|---|
| Acrylat A [g] | 49,85 | | 36,85 | |
| Methacrylat B [g] | | 49,85 | | |
| Methacrylat D [g] | | | | 48,85 |
| Ethoxyliertes Bisphenol-A-dimethacrylat [g] (SR 348, Sartomer) | 26,0 | 26,0 | 6,0 | |
| Ethoxyliertes Bisphenol-A-diacrylat [g] (SR 349, Sartomer) | | | 26,0 | 25,0 |
| Trimethylolpropantrimethacrylat (SR 350, Sartomer) [g] | 14,0 | 14,0 | 6,0 | |
| Trimethylolpropantriacrylat (SR 351, Sartomer) [g] | | | 14,0 | 12,0 |
| Phenoxyethylacrylat (SR 339, Sartomer) [g] | 6,0 | 6,0 | 6,0 | 5,0 |
| 1-Hydroxycyclohexylphenylketon [g] | 4,0 | 4,0 | 5,0 | 4,0 |
| N-Vinylpyrrolidon [g] | | | | 5,0 |
| Hydrochinonmonomethylether [g] | 0,15 | 0,15 | 0,15 | 0,15 |
| Viskosität η der flüssigen Mischung bei 30 °C [mPa·s] | 631 | 578 | 451 | 302 |
| Eigenschaften der Grünlinge: | | | | |
| Elastizitätsmodul [N/mm²] | 16,2 | 20,4 | 35,8 | |
| Zugfestigkeit σₘₐₓ [N/mm²] | 1,31 | 1,56 | 2,70 | |
| Bruchdehnung ε [%] | 10,2 | 20,4 | 12,5 | |
| Eigenschaften der ausgehärteten Formkörper: | | | | |
| Elastizitätsmodul [N/mm²] | 1610 | 1734 | 1660 | 251,3 |
| Zugfestigkeit σₘₐₓ [N/mm²] | 32,8 | 35,0 | 32,0 | 9,8 |
| Bruchdehnung ε [%] | 7,2 | 4,1 | 5,0 | 11,0 |

**Tabelle 2:**

| Beispiel | 5 | 6 | 7 | 8 | 9 | 10 |
|---|---|---|---|---|---|---|
| Acrylat A [g] | 9,0 | | 30,0 | | 19,0 | |
| Methacrylat B [g] | | 9,0 | | 30.0 | | 19.0 |
| Ethoxyliertes Bisphenol-A-dimethacrylat (SR 348, Sartomer) [g] | 29,0 | 29,0 | 5,0 | 5,0 | 29,0 | 29,0 |
| Ethoxyliertes Bisphenol-A-diacrylat [g] (SR 349, Sartomer) | | | 20,0 | 20,0 | | |
| Polyethylenglykol-400-diacrylat [g] (SR 344, Sartomer) | 14,0 | 14,0 | | | 14,0 | 14,0 |
| Neopentylglykoldiacrylat | 7,0 | 7,0 | | | 7,0 | 7,0 |
| Trimethylolpropantriacrylat [g] (SR 351, Sartomer) | | | 12,0 | 12,0 | | |
| Phenoxyethylacrylat [g] | 1,0 | 1,0 | 5,0 | 5,0 | 1,0 | 1,0 |
| 1-Hydroxycyclohexylphenylketon [g] | 5,0 | 5,0 | 5,0 | 5,0 | 5,0 | 5,0 |
| aliphatisches Urethanacrylat [g] (SR 9640, Sartomer, MG: 1300, Viskosität bei 60 °C: 18000 mPa·s) | 35,0 | 35,0 | 23,0 | 23,0 | 25,0 | 25,0 |
| Viskosität η der flüssigen Mischung bei 30 °C [mPa · s] | 1490 | 1600 | 2250 | 2120 | 1010 | 925 |
| Eigenschaften der Grünlinge: | | | | | | |
| Elastizitätsmodul [N/mm²] | 23,9 | 59,4 | 52,2 | 36,4 | 19,6 | 27,3 |
| Zugfestigkeit σₘₐₓ [N/mm²] | 3,7 | 3,8 | 3,7 | 3,9 | 2,9 | 3,3 |
| Bruchdehnung ε [%] | 22,8 | 17,4 | 13,6 | 20,0 | 19,9 | 18,8 |
| Eigenschaften der ausgehärteten Formkörper: | | | | | | |
| Elastizitätsmodul [N/mm²] | 729 | 948 | 772 | 1340 | 941 | 1102 |
| Zugfestigkeit σₘₐₓ [N/mm²] | 26,6 | 27,8 | 18,7 | 24,8 | 25,8 | 30,0 |
| Bruchdehnung ε [%] | 21,0 | 17,4 | 7,5 | 6,3 | 13,5 | 16,0 |

## Patentansprüche

1. Verbindungen der Formeln (Ia) und (Ib) 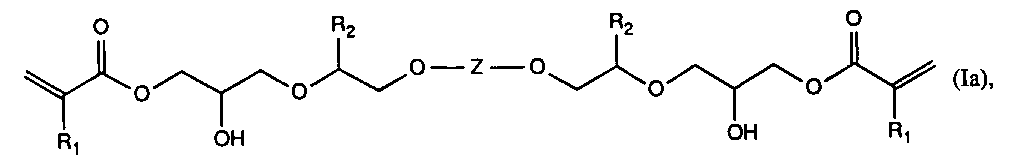 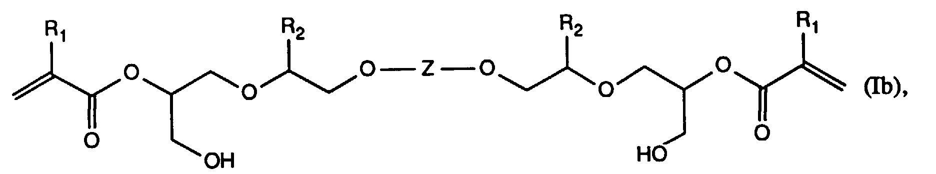 worin die Reste R₁ unabhägig voneinander Wasserstoff oder Methyl bedeuten,
R₂ für eine C₁-C₂₀-Alkylgruppe, die gegebenenfalls durch eine oder mehrere Hydroxygruppen, C₆-C₁₄-Arylgruppen oder Halogenatome substituiert ist, eine unsubstituierte oder durch eine oder mehrere C₁-C₆-Alkylgruppen, Hydroxygruppen oder Halogenatome substituierte Phenylgruppe oder für einen Rest der Formel -CH₂-OR₃ steht, worin R₃ eine C₁-C₂₀-Alkylgruppe, die gegebenenfalls durch eine oder mehrere Hydroxygruppen, C₆-C₁₄-Arylgruppen oder Halogenatome substituiert ist, eine unsubstituierte oder durch eine oder mehrere C₁-C₆-Alkylgruppen, Hydroxygruppen oder Halogenatome substituierte Phenylgruppe, eine C₂-C₆-Alkenylgruppe, eine C₂-C₂₀-Acylgruppe oder eine unsubstituierte oder durch eine oder mehrere C₁-C₆-Alkylgruppen, Hydroxygruppen oder Halogenatome substituierte Cyclohexylcarbonylgruppe bedeutet,
Z eine Gruppe der Formeln (IIa)-(IIe) ist, 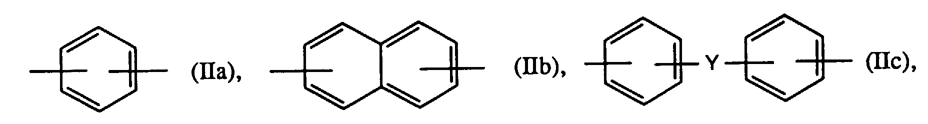 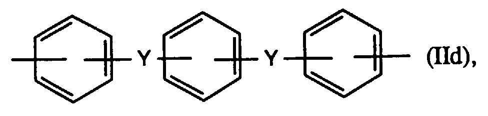 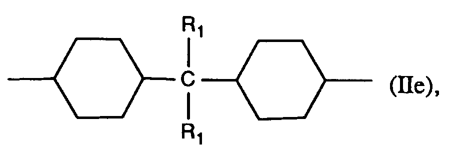 worin Y für eine direkte Bindung, C₁-C₆-Alkylen, -S-, -O-, -SO-, -SO₂- oder -CO- steht und R₁ Wasserstoff oder Methyl bedeutet und worin die aromatischen bzw. cycloaliphatischen Ringe der Formeln (IIa)-(IIe) unsubstituiert oder durch eine oder mehrere C₁-C₆-Alkylgruppen, Chlor- oder Bromatome substituiert sind.

2. Verbindungen der Formeln (Ia) und (Ib) gemäss Anspruch 1, worin R₂ für C₁-C₂₀-Alkyl, Phenyl, C₁-C₂₀-Alkoxymethyl, Phenoxymethyl oder Cyclohexylcarbonyloxymethyl steht

3. Verbindungen der Formeln (Ia) und (Ib) gemäss Anspruch 1, worin R₂ für n-Butyl, Phenyl, n-Butoxymethyl, Phenoxymethyl oder Cyclohexylcarbonyloxymethyl steht.

4. Verbindungen der Formeln (Ia) und (Ib) gemäss Anspruch 1, worin R₂ n-Butoxymethyl ist.

5. Verbindungen der Formeln (Ia) und (Ib) gemäss Anspruch 1, worin Z für eine Gruppe der Formeln (IIc) oder (IIe) steht.

6. Verbindungen der Formeln (Ia) und (Ib) gemäss Anspruch 1, worin Z für

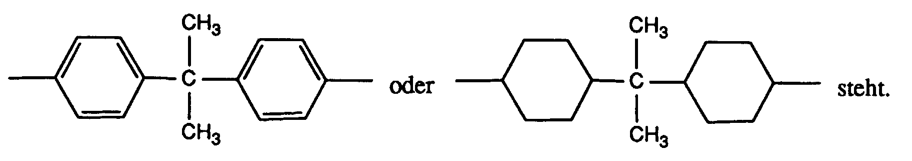

7. Verfahren zur Herstellung von Verbindungen der Formeln (Ia) und (Ib) gemäss Anspruch 1, dadurch gekennzeichnet, dass man einen Diglycidylether der Formel (III) 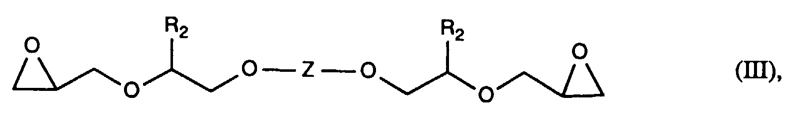 worin R₂ und Z die in Anspruch 1 angegebene Bedeutung haben, in an sich bekannter Weise mit Acrylsäure oder Methacrylsäure umsetzt.

8. Photoempfindliche Zusammensetzung enthaltend
(a) 5-65 Gew.-% einer Verbindung der Formel (Ia) oder (Ib) gemäss Anspruch 1,
(b) 15-70 Gew.-% eines oder mehrerer von der Verbindung der Formel (Ia) oder (Ib) verschiedenen bifunktionellen Acrylate oder Methacrylate mit einem Molekulargewicht zwischen 150 und 450,
(c) 0-40 Gew.-% eines oder mehrerer monomerer polyfunktioneller Acrylate oder Methacrylate mit einer Funktionalität von mindestens 3 und einem Molekulargewicht von höchstens 600,
(d) 0-10 Gew.-% mindestens eines monofunktionellen Acrylats oder Methacrylats,
(e) 0-10 Gew.-% N-Vinylpyrrolidon oder N-Vinylcaprolactam,
(f) 2-10 Gew.-% mindestens eines Photoinitiators und
(g) 0-60 Gew.-% mindestens eines Urethanacrylats oder -methacrylats mit einer Funktionalität von 2-4 und einem Molekulargewicht von 500-10000,
wobei die Summe der Anteile der Komponenten (a) bis (g) zusammen 100 Gew.-% beträgt.

9. Zusammensetzung gemäss Anspruch 8 enthaltend als Komponente (b) ein Diacrylat oder Dimethacrylat von ethoxyliertem Bisphenol A.

10. Zusammensetzung gemäss Anspruch 8 enthaltend als Komponente (c) Trimethylolpropantriacrylat oder Trimethylolpropantrimethacrylat.

11. Zusammensetzung gemäss Anspruch 8 enthaltend als Komponente (d) Phenoxyethylacrylat.

12. Zusammensetzung gemäss Anspruch 8 enthaltend als Komponente (f) 1-Hydroxycyclohexylphenylketon.

13. Verfahren zur Herstellung von dreidimensionalen Gegenständen aus einer photoempfindlichen Zusammensetzung gemäss Anspruch 8 mittels Stereolithographie wobei die Oberfläche einer Schicht aus der erfindungsgemässen Zusammensetzung ganzflächig oder in einem vorbestimmten Muster mit UV- oder VIS-Licht bestrahlt wird, so dass sich in den bestrahlten Bereichen eine Schicht in einer gewünschten Schichtdicke verfestigt, dann eine neue Schicht aus den erfindungsgemässen Gemischen auf der verfestigten Schicht gebildet wird, die ebenfalls ganzflächig oder in einem vorbestimmten Muster bestrahlt wird und wobei durch wiederholtes Beschichten und Bestrahlen dreidimensionale Gegenstände aus mehreren aneinander haftenden, verfestigten Schichten erhalten werden.

## Claims

1. A compound of the formula (Ia) or (Ib) 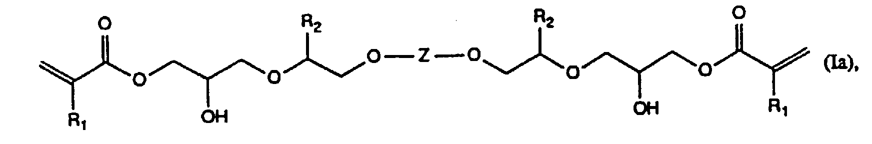 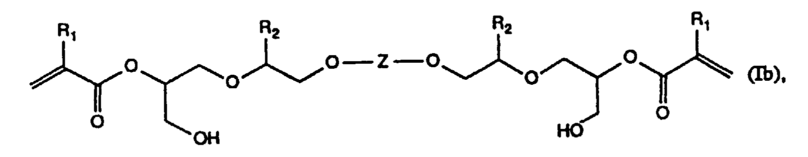 wherein the radicals R₁ independently of one another are hydrogen or methyl, R₂ is an unsubstituted C₁-C₂₀alkyl group or a C₁-C₂₀alkyl group which is substituted by one or more hydroxyl groups, C₆-C₁₄aryl groups or halogen atoms, an unsubstituted phenyl group or a phenyl group which is substituted by one or more C₁-C₆alkyl groups, hydroxyl groups or halogen atoms, or is a radical of formula -CH₂-OR₃, wherein R₃ is an unsubstituted C₁-C₂₀alkyl group or a C₁-C₂₀alkyl group which is substituted by one or more hydroxyl groups, C₆-C₁₄aryl groups or halogen atoms, an unsubstituted phenyl group or a phenyl group which is substituted by one or more C₁-C₆alkyl groups, hydroxyl groups or halogen atoms, or is a C₂-C₆alkenyl group, a C₂-C₂₀acyl group or an unsubstituted cyclohexylcarbonyl group or a cyclohexylcarbonyl group which is substituted by one or more C₁-C₆alkyl groups, hydroxyl groups or halogen atoms,
Z is a group of formulae (IIa)-(IIe), 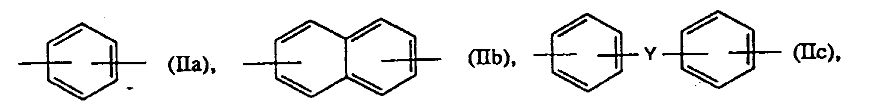 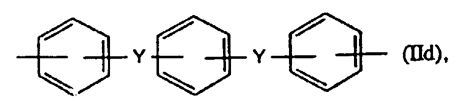 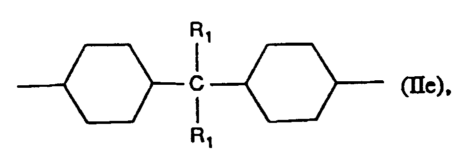 wherein Y is a direct bond, C₁-C₆alkylene, -S-, -O-, -SO-, -SO₂- or -CO-, and R₁ is hydrogen or methyl, and wherein the aromatic and cycloaliphatic rings of formulae (IIa)-(IIe) are unsubstituted or substituted by one or more C₁-C₆alkyl groups, chlorine or bromine atoms.

2. A compound of formula (Ia) or (Ib) according to claim 1, wherein R₂ is C₁-C₂₀alkyl, phenyl, C₁-C₂₀alkoxymethyl, phenoxymethyl or cyclohexylcarbonyloxymethyl.

3. A compound of formula (Ia) or (Ib) according to claim 1, wherein R₂ is n-butyl, phenyl, n-butoxymethyl, phenoxymethyl or cyclohexylcarbonyloxymethyl.

4. A compound of formula (Ia) or (Ib) according to claim 1, wherein R₂ is n-butoxymethyl.

5. A compound of formula (Ia) or (Ib) according to claim 1, wherein Z is a group of formula (IIc) or (IIe).

6. A compound of formula (Ia) or (Ib), according to claim 1, wherein Z is

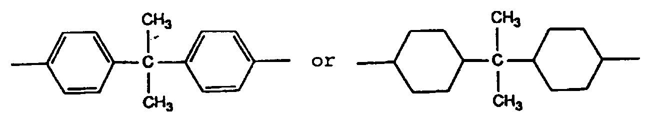

7. A process for the preparation of a compound of formula (Ia) or (Ib) according to claim 1, which comprises reacting a diglycidyl ether of formula (III) 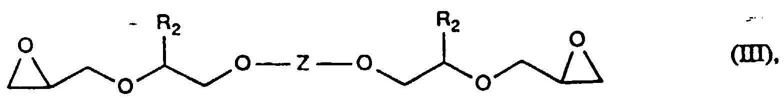 wherein R₂ and Z have the meaning given in claim 1, in a manner known per se with acrylic or methacrylic acid.

8. A photosensitive composition comprising
(a) 5-65 % by weight of a compound of formula (Ia) or (Ib) according to claim 1,
(b) 15-70 % by weight of one or more bifunctional acrylates or methacrylates having a molecular weight of between 150 and 450 and differing from the compound of formula (Ia) or (Ib),
(c) 0-40 % by weight of one or more monomeric polyfunctional acrylates or methacrylates having a functionality of not less than 3 and a molecular weight of not more than 600,
(d) 0-10 % by weight of at least one monofunctional acrylate or methacrylate,
(e) 0-10 % by weight of N-vinylpyrrolidone or N-vinylcaprolactam,
(f) 2-10 % by weight of at least one photoinitiator, and
(g) 0-60 % by weight of at least one urethane acrylate or methacrylate having a functionality of 2-4 and a molecular weight of 500-10 000, the sum of the amounts of components (a) to (g) together being 100 % by weight.

9. A composition according to claim 8, comprising as component (b) a diacrylate or dimethacrylate of ethoxylated bisphenol A.

10. A composition according to claim 8, comprising as component (c) trimethylolpropane triacrylate or trimethylolpropane trimethacrylate.

11. A composition according to claim 8, comprising as component (d) phenoxyethyl acrylate.

12. A composition according to claim 8, comprising as component (f) 1-hydroxycyclohexyl phenyl ketone.

13. A process for the production of three-dimensional objects from a photosensitive composition according to claim 8 by stereolithography, wherein the surface of a layer of the novel composition is irradiated over its entire area or in a predetermined pattern with UV or VIS light, such that within the irradiated areas a layer solidifies in a desired layer thickness, then a new layer of the novel mixtures is formed on the solidified layer, which is likewise irradiated over its entire area or in a predetermined pattern, and such that three-dimensional objects formed from a plurality of solidified layers which adhere to one another are obtained by repeated coating and irradiation.

## Revendications

1. Composés de formules (Ia) et (Ib) 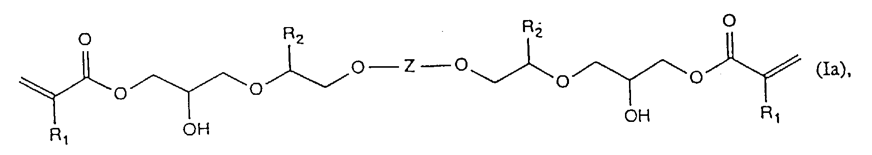 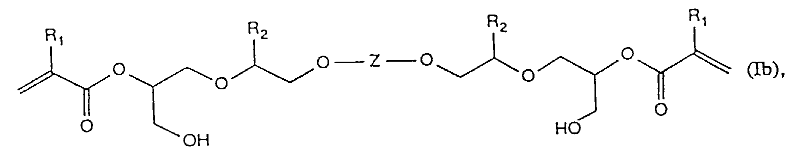 dans lesquels les radicaux R₁ indépendamment les uns des autres représentent l'hydrogène ou méthyle, R₂ représente un groupe alkyle en C₁-C₂₀, qui peut être éventuellement substitué par un ou plusieurs groupes hydroxyle, aryle en C₆-C₁₄ ou atomes d'halogènes, un groupe phényle non substitué ou substitué une ou plusieurs fois par des groupes alkyle en C₁-C₆, hydroxyle ou atomes d'halgènes ou un radical de formule -CH₂-OR₃, dans laquelle R₃ représente un groupe alkyle en C₁-C₂₀, qui est éventuellement substitué par un ou plusieurs groupes hydroxy, groupe aryle en C₆-C₁₄ ou atome d'halogène, un groupe phényle non substitué ou substitué une ou plusieurs fois par des groupes alkyle en C₁-C₆, des groupes hydroxy ou des atomes d'halogènes, un groupe alcényle en C₂-C₆, un groupe acyle en C₂-C₂₀ ou un groupe cyclohexyl-carbonyle non substitué ou substitué par un ou plusieurs groupes alkyle en C₁-C₆, hydroxyle ou atomes d'halogènes,
Z est un groupe de formules (IIa) à (IIe) 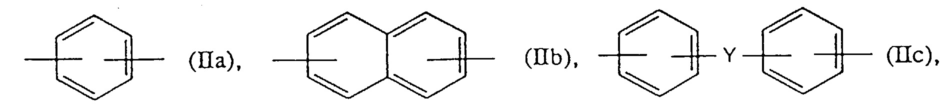 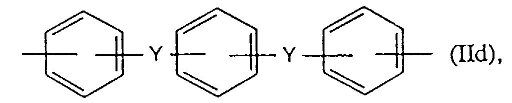 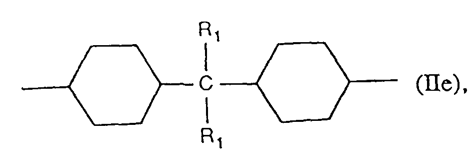 dans lesquelles Y représente une liaison directe, alkylène en C₁-C₆, -S-, -O-, -SO-, -SO₂- ou -CO- et R₁ représente l'hydrogène ou méthyle, et dans lesquelles les cycles aromatiques respectivement cycloaliphatiques de formules (IIa) à (IIe) sont non substitués ou substitués par un ou plusieurs groupes alkyles en C₁-C₆, atomes de chlore ou de brome.

2. Composés de formules (Ia) et (Ib) selon la revendication 1, dans lesquels R₂ représente alkyle en C₁-C₂₀, phényle, alcoxyméthyle en C₁-C₂₀, phénoxyméthyle ou cyclohexylcarbonyloxyméthyle.

3. Composés de formules (Ia) et (Ib) selon la revendication 1 dans lesquels R₂ représente n-butyle, phényle, n-butoxyméthyle, phénoxyméthyle ou cyclohexylcarbonyloxyméthyle.

4. Composés de formules (Ia) et (Ib) selon la revendication 1, dans lesquels R₂ représente n-butoxyméthyle.

5. Composés de formules (Ia) et (Ib) selon la revendication 1, dans lesquels Z représente un groupe de formules (IIc) ou (IIe).

6. Composés de formules (Ia) et (Ib) selon la revendication 1, dans lesquels Z représente

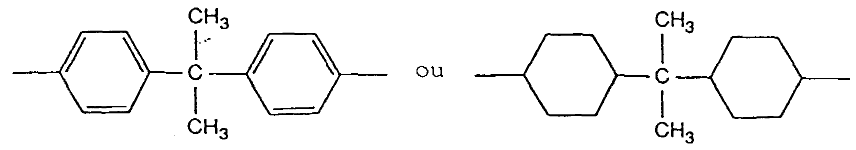

7. Procédé pour la préparation de composés de formules (Ia) et (Ib) selon la revendication 1 caractérisés en ce qu'on fait réagir un éther diglycidylique de formule (III) 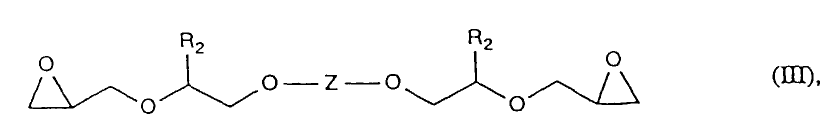 dans laquelle R₂ et Z ont la signification donnée dans la revendication 1, de façon connue en soi, avec l'acide méthacrylique ou l'acide acrylique.

8. Composition photosensible comprenant :
(a) de 5 à 65 % en poids d'un composé de formule (Ia) ou (Ib) selon la revendication 1,
(b) de 15 à 70 % en poids d'un ou plusieurs composés de formule (Ia) ou (Ib) de différents méthacrylates ou acrylates bifonctionnels ayant un poids moléculaire compris entre 150 et 450,
(c) de 0 à 40 % en poids d'un ou plusieurs méthacrylates ou acrylates monomères polyfonctionnels ayant une fonctionnalité d'au moins 3 et un poids moléculare de tout au plus 600,
(d) de 0 à 10 % en poids d'au moins un méthacrylate ou acrylate monofonctionnel,
(e) de 0 à 10 % en poids de N-vinylpyrrolidone ou de N-vinylcaprolactame,
(f) de 2 à 10 % en poids d'au moins un photoamorceur, et
(g) de 0 à 60 % en poids d'au moins un uréthanne-acrylate ou -méthacrylate ayant une fonctionnalité de 2 à 4 et un poids moléculaire de 500 à 10 000,
la somme des taux en constituants (a) à (g), ensemble, étant de 100%.

9. Composition selon la revendication 8, contenant en tant que composant (b), un diacrylate ou diméthacrylate du bisphénol A éthoxylé.

10. Composition selon la revendication 8, contenant en tant que constituant (c), le triacrylate du triméthylolpropane ou le triméthacrylate du triméthylolpropane.

11. Composition selon la revendication 8, contenant en tant que composant (d), l'acrylate de phénoxyéthyle.

12. Composition selon la revendication 8, contenant en tant que constituant (f) la 1-hydroxycyclohexylphénylcétone.

13. Procédé pour la préparation d'objets tridimensionnels d'un mélange photosensible selon la revendication 8, à l'aide de stéréolithographie en irradiant la surface d'une couche de la composition conforme à l'invention, sur la totalité de sa surface ou selon un modèle prédéterminé, par la lumière UV ou VIS, de façon telle à ce qu'il se solidifie sur les zones irradirées une couche d'une épaisseur désirée, puis il se forme une nouvelle couche des mélanges conformes à l'invention sur la couche solidifiée, que l'on irradie également sur la totalité de la surface ou selon un modèle prédéterminé, en obtenant par enduction et irradiation répétées des objets tridimensionnels constitués de plusieurs couches solidifiées adhérentes l'une sur l'autre.
